Europäisches Patentamt

European Patent Office

Office européen des brevets

(11) Veröffentlichungsnummer: **0 022 977**
**B1**

(12) **EUROPÄISCHE PATENTSCHRIFT**

(45) Veröffentlichungstag der Patentschrift: **19.12.84**

(51) Int. Cl.³: **A 61 M 5/32**

(21) Anmeldenummer: **80103882.9**

(22) Anmeldetag: **08.07.80**

(54) **Vorrichtung für Injektionsspritzen.**

(30) Priorität: **20.07.79 DE 2929425**

(43) Veröffentlichungstag der Anmeldung:
**28.01.81 Patentblatt 81/04**

(45) Bekanntmachung des Hinweises auf die
Patenterteilung:
**19.12.84 Patentblatt 84/51**

(84) Benannte Vertragsstaaten:
**AT BE CH DE FR GB IT LI LU NL SE**

(56) Entgegenhaltungen:
**DE-A-1 166 419**
**US-A-3 820 542**

(73) Patentinhaber: **Kling, Lothar**
**Bierstädter Höhe 24**
**D-6200 Wiesbaden (DE)**

(72) Erfinder: **Kling, Lothar**
**Bierstädter Höhe 24**
**D-6200 Wiesbaden (DE)**

**Beschreibung**

Die Erfindung betrifft eine Vorrichtung für Injektionsspritzen zur intramuskulären und subkutanen Injektion in der Human- und Veterinärmedizin, wobei unter Verwendung coaxial ineinander verschiebbarer Teile, einschließlich einem in Verlängerung und teilweiser Umfassung des Spritzenkörpers unten zur Injektionsstelle hin offenen Applikator zum Ansetzen bzw. Applizieren an den zu behandelnden Körper, wobei durch Druck auf den Spritzenkörper dieser samt Nadel unter Überwindung eines Klemmelementes zum raschen schmerzfreien Einstechen schlagartig nach vorne bewegbar ist bis zum Erreichen eines die Verschiebung aufhaltenden Anschlagelementes.

Eine derartige Vorrichtung ist aus der US—A—3 820 542 sowie DE—B—11 66 419 bekanntgeworden.

In der US—A—3 820 542 ist eine speziell ausgebildete Spritze beschrieben (deren Einzelheiten hier nicht interessieren), wobei das obere Teil des zylindrisch geformten Spritzenkörpers von einem ebenfalls zylindrischen und im wesentlichen hülsenartigen Applikator umfaßt wird. Der Spritzenkörper ist im unteren Bereich, wo die Nadel austritt, erweitert, ebenso der Applikator, in den der Spritzenkörper von unten eingeführt werden muß und nach Verdrehen um 90° vermittels korrespondierender Klemmelemente an Spritzenkörper und Applikator in einer oberen Ausgangsstellung gehaltert werden kann. Nach Überwindung dieser Arretierung schnellt der Spritzenkörper mit Kolben und Nadel nach unten und wird von einem am unteren Rand des Applikators befindlichen, nach innen sich erstreckenden Flansch aufgehalten.

Abgesehen davon, daß dieser Applikator nicht für übliche Kunststoff-Injektionsspritzen, insbesondere Insulinspritzen, geeignet ist, erscheint der Aufbau aus Metall bei der gegebenen recht komplizierten Gestaltung sehr kostspielig. Als besonders unerwünscht muß die zeitaufwendige und einige Geschicklichkeit erfordernde Montage des Spritzenkörpers in dem Applikator angesehen werden. Im übrigen führt das zwar weiche Ansetzen des Applikators auf der Haut des Patienten vermöge des unten verbreiterten Randes zu einer aus heutiger Sicht unewünschten Konzentration des Patienten auf den Einstichschmerz.

Auch in der vorgenannten DE—B—11 66 419 wird mehrfach betont, daß der dort als Ansetzstütze bezeichnete Applikator so ausgebildet werden sollte, daß er möglichst schmerzfrei an die betreffende Stelle des Körpers des Patienten angedrückt werden kann. Daher wird dort ein Randwulst vorgesehen. Im übrigen ist die beschriebene Vorrichtung mehrteilig ausgebildet und die vorgeschlagenen Klemmelemente erscheinen teilweise keine zuverlässig reproduzierbare Haltekraft zu gewährleisten, so daß teils mit zu wenig, in anderen Fällen mit zu viel Tempo und Kraft eingestochen wird.

Zwar wird hier schon Kunststoff als Material empfohlen, doch führt die Verwendung mehrerer coaxial ineinander verschiebbarer Teile und der Übergang von einem dem Spritzenkörper umfassenden Applikator auf eine nur die Kanülenhalterung umgreifende Vorrichtung mit kleinerem Durchmesser von einer einfachen und ausreichend form- und lage-stabilen Lösung weg.

Dabei ist an sich erwünscht, die Haut an der Injektionsstelle zu spanne, um einen schnellen und damit schmerzlosen Durchstich ermöglichen zu können, die Injektionsnadel schnell durch die Haut zu treiben, einen Ablenkungsdruck oder -schmerz zu erzielen und insbesondere bei der Eigeninjektion eine psychische Angstschwelle vor dem Stich mit der Injektionsnadel zu überwinden.

Im übrigen ist bekannt, daß die Schmerzfreiheit einer Injektion weitgehend von der Geschicklichkeit des Arztes, der Krankenschwester oder im Falle der Selbstinjizierung von der Geschicklichkeit der betreffenden Person abhängt. Dieses Problem ergibt sich insbesondere bei Personen, die an Diabetes erkrankt sind und auf zwei oder mehrere Insulinspritzen pro Tag angewiesen sind.

Der Erfindung liegt daher die Aufgabe zugrunde, die genannten Nachteile des Standes der Technik zu beseitigen und eine Vorrichtung für die Applikation einer Injectionsspritze zu schaffen, die aus möglichst wenig Teilen bestehend einfach, zuverlässig und angenehm handhabbar und insbesondere auf moderne Einweg-Kunststoffspritzen anwendbar sowie ggf. selbst als Massenartikel kostengünstig herstellbar ist.

Die vorgenannte Aufgabe wird bei einer Vorrichtung der eingangs genannten Art gemäß der Erfindung dadurch gelöst, daß der mit einem Spritzenkörper von gelichmäßigem Umfang zu kombinierende Applikator aus einer einteiligen im wesentlichen rohrförmigen Kunststoffhülse besteht mit einem unteren für die Erzeugung eines Ablenkungsschmerzes geeigneten Rand und einem diesem entgegegesetzt liegenden oberen Bereich, dessen Innendurchmesser etwas größer ist als der Außendurchmesser des von oben einzuführenden Spritzenkörpers, und daß die Hülse weiterhin als Klemm- und Anschlagselement an ihre Innenwand angeformte Erhebungen, insbesondere Wulste, als Querschnittsverengung aufweist in einem vorgegebenen axialen Abstand voneinander, der der gewünschten Injektionstiefe zuzüglich einem Sicherheitsabstand zwischen Nadelspitze und unteren Hülsenrang in Ausgangsstellung entspricht, wobei das Anschlagselement zwischen Klemmelement und unterem Hülsenrand angeordnet ist.

Mit anderen Worten gibt also das im Inneren der Hülse befindliche Klemmelement die Fortbewegung des in die Hülse von oben einschiebbaren Spritzenkörpers mit der Nadel bei Erreichen eines bestimmten Druckes frei. Dabei sind die Länge der Hülse und der Abstand des Klemmelementes vom unteren Hülsenrand so bemessen, daß die Injektionsnadel in der Ausgangsstellung zur Gänze in der Hülse liegt und daß nach der Freigabe der Injektionsspritze durch das Klemmelement und deren Fortbewegung die Injektionsnadel aus der Hülse austritt und die gewünschte Injektionstiefe außerhalb der Hülse erreichbar ist.

Die weitere vorteilhafte Ausgestaltung der Erfindung ergibt sich aus den Merkmalen der Unteransprüche.

Mit der Vorrichtung gemäß der Erfindung ist es gelungen, eine komplexe Aufgabenstellung zu erfüllen und einen bisher sehr umständlich verwirklichten Ablauf—Einsetzen der Spritze/Klemmstellung herstellen/Überwindung der Klemmstellung/Vorschnellen/Aufhalten des Spritzenkörpers—mit einer überraschend einfach ausgestaltbaren und leicht als Massenartikel z.B. im Spritzgußverfahren herstellbaren einteiligen Vorrichtung zuverlässig zu ermöglichen.

Im einzelnen bestehen, die mit der Erfindung erzielbaren Vorteile darüber hinaus insbesondere im Folgenden:

— unkomplizierte, rasche Handhabung;
— Erleichterung des gesamtem Vorganges der Injektion, besonders bei Selbstinjektion und in Situationen, wo nur eine Hand hierfür benutzt werden kann;
— Minimierung des Einstichschmerzes durch mäßigen Ablenkungsschmerz. Spannung der Haut und rasches Einschnellen der Injektionsnadel;
— Im Falle der Benutzung eines undurchsichtigen Materials für die Hülse ist die Injektionsnadel unsichtbar, was die Angst vor der Nadel verringert und damit auch dem Arzt die Verordnung der Selbstinjektion erleichtert;
— die Vorrichtung kann bei fertigverpackten Injektionsspritzen das bisher übliche Schutzröhrchen für die Injektionsnadel ersetzen und damit die Herstellkosten hierfür einsparen.

Im folgenden wird die Erfindung anhand eines Ausführungsbeispieles näher erläutert, aus dem sich weitere Vorteile und Merkmale der Erfindung ergeben. Es zeigen:

Figure 1—eine perspektivische Ansicht einer Ausführungsform der Erfindung;

Figur 2—einen Schnitt durch die in Figur 1 gezeigte Ausführungsform;

Figur 3—eine gegenüber Figur 1 geringfügig abgewandelte Ausführungsform der Erfindung;

Figur 4a bis 4e—schematisch den Phasenablauf während des Injektionsvorganges, wobei die Injektionsspritze in ihrer Stellung innerhalb der Vorrichtung während der einzelnen Phasen des Injektionsvorganges dargestellt ist;

Figur 5—eine mit der Injektionsspritze integrierte Vorrichtung nach der Erfindung;

Figur 6—eine andere Ausführungsform der Vorrichtung nach der Erfindung, und

Figur 7—eine perspektivische Ansicht einer weiteren Ausführungsform der Erfindung, die einen schrägen Hülsenrand aufweist.

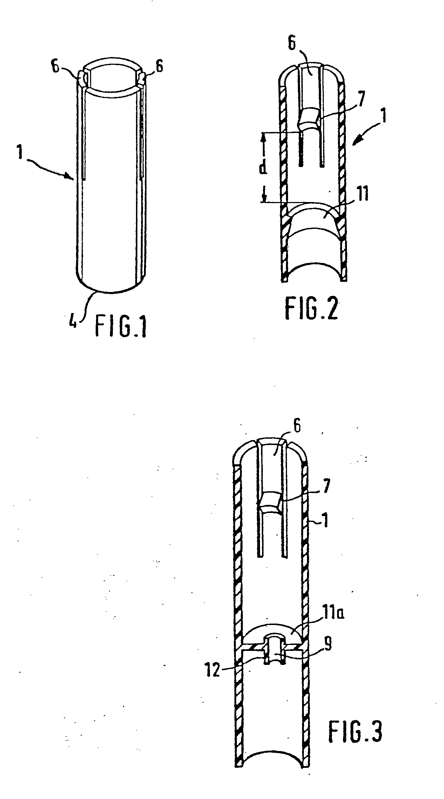

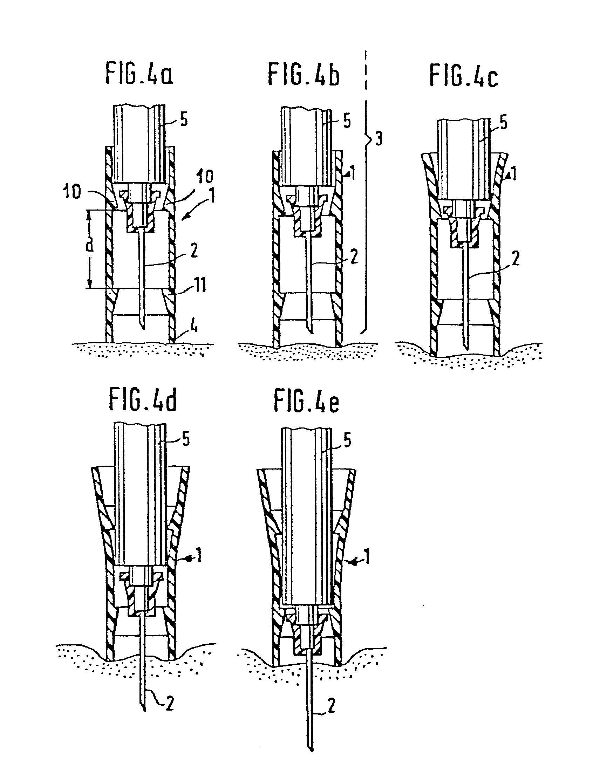

Figur 1 zeigt eine perspektivische Ansicht einer Ausführungsform der Vorrichtung, die als rohrförmige Hülse 1 ausgebildet ist, welche in ihrer Umfangsfläche zwei Zungen 6, 6' aufweist, die sich diametral gegenüberliegen. Der Innendurchmesser der rohrförmigen Hülse 1 ist etwas größer als der Außendurchmesser einer zugehörigen Injektionsspritze 3, wie dies aus den Figuren 4a bis 4e hervorgeht. Diese Ausführungsform besitzt einen unteren Hülsenrand 4, der senkrecht zur Längsrichtung der Hülse 1 verläuft.

In Figur 2 ist im schmitt in perspektivischer Darstellung eine weitere Ausführungsform der Hülse 1 gezeigt, die eine einzige oder zwei Zungen 6 in ihrer Umfangsfläche besitzt. Die Zunge 6 ist mit einer Nase 7 versehen, gegen welche die nicht dargestellte Injektionsspritze 3 zu Beginn des Injektionsvorganges, der nachfolgend anhand der Figuren 4a bis 4e näher beschrieben werden wird, anliegt. Im Abstand d von dem interen Rand der Nase 7 befindet sich ein Anschlagelement 11, das mit leicht konischem Verlauf in das Innere der Hülse 1 gerichtet ist und aus einem ringförmigen Wulst gebildet ist.

In Figur 3 ist in perspektivischer Schnittansicht eine weitere Ausführungsform der Hülse 1 dargestellt, die eine einzige Zunge 6 in der Umfangsfläche besitzt. Als Anschlag ist ein Zwischenboden 11a vorgesehen, der eine zentrische Öffnung 9 zum Passieren der Injektionsnadel 2 aufweist. Die zentrische Öffnung 9 ist von einer koaxialen Hülse 12 umgeben. Diese ist so angeordnet, daß sie beim Aufstecken der Hülse 1 auf den Spritzenkörper der Injektionsspritze die Injektionsnadel umgibt.

Als Material für die Hülse 1 wird bevorzugt nichttransparenter Kunststoff verwendet, wobei die Wandstärke der Hülse 0,5 bis 1,5 mm betragen kann. Es wird ein Kunststoff verwendet, der für das Klemmelement 10 eine bestimmte Biegesteifheit und eine Mindestelastizität besitzt, die sicherstellen, daß die Injektionsspritze 3 erst nach Überwindung der elastischen Klemmkraft des Elements 11 vorwärts bewegt werden kann. Die Länge der Hülse 1 und der Abstand des Klemmelements 10 vom unteren Hülsenrand 4 sind so bemessen, daß die Injektionsnadel 2 zu Beginn des Injektionsvorganges zur Gänze in der Hülse 1 liegt. Nach der Freigabe der Injektionsspritze 3 durch Überschreiten des erforderlichen Druckes auf das Klemmelement 10 tritt die Injektionsnadel 2 aus der Hülse 1 aus und sticht in die Haut der jeweiligen Person, die die Injektion empfängt, ein. Der Abstand d zwischen dem Klemmelement 10 und dem Anschlagelement 11 ist so bemessen, daß er der gewünschten Injektionstiefe zuzüglich einem Sicherheitsabstand zwischen der Nadelspitze und dem Hülsenrand 4 entspricht.

Die verschiedenen Phasen des Vorganges werden im folgenden anhand der Figuren 4a bis 4e beschrieben. Die Hülse 1 entspricht der Ausführungsform nach Figur 1 mit den beiden Zungen 6, 6' in der Umfangsfläche der Hülse 1. Die Injektionsspritze 3 ist nur teilweise dargestellt, der obere Teil der Injektionsspritze ist weggelassen, da er für die Erläuterung des Injektionsvorganges unwesentlich ist. Auf den Spritzenkörper 5 ist die metallische Injektionsnadel 2 aufgesetzt, die in Figur 4a zur Gänze innerhalb der Hülse 1 liegt. Das Anschlagelement 11 in den Figuren 4a bis 4e ist ein umlaufender, sich konisch nach innen auswölbender Ring.

In Figur 4a ist die Injektionsspritze 3 mit der Hülse 1 ohne Druckausübung auf die Haut aufgesetzt. Der auf die Injektionsspritze 3 ausgeübte Druck steigert sich von der Darstellung in Figur 4b allmählich und erreicht seinen Höchstwert in der in Figur 4e gezeigten Phase.

In Figur 4b wird zunächst durch Druckausübung auf das obere, nicht dargestellte Ende der Injektionsspritze 3 die Hauptoberfläche gespannt und wölbt sich über den unteren Hülsenrand 4 a leicht in das Innere der Hülse 1.

Durch verstärkten Druck auf as hintere Ende der Injektionsspritze 3 wird die Druckspannung auf die Haut entlang dem unteren Hülsenrand 4 verstärkt, wodurch ein sogenannter Ablenkungsdruck bzw. Ablenkungsschmerz entlang dem Hülsenrand 4 in der Hautoberfläche auftritt. Dieser Ablenkungsschmerz wird jedoch von der jeweiligen Person erfahrungsgemäß als nur sehr gering empfunden. Die beiden Zungen die gleichfalls jeweils mit einer nach innen vorspringenden Nase ausgerüstet sind, werden durch den Spritzenkörper 5 auseinander gebogen, jedoch wird die Klemmkraft des Klemmelements 10 noch nicht durch den auf die Injektionsspritze 3 ausgeübten Druck überschritten. Dies tritt erst in der in Figur 4d gezeigten Phase auf, in der der Spritzenkörper 5 mit seiner Unterkante zwischen dem Klemmelement 10 und dem Anschlagelement 11 liegt. In dieser Phase ist die Injektionsnadel 2 nahezu schmerzfrei in die Haut eingedrungen.

Figur 4e zeigt die Endphase des Injektionsvorgangs, in der das Anschlagelement 11, beispielsweise als Stoppring in der Kunststoffhülse ausgeformt, den Spritzenkörper 5 mit seiner Unterkante aufhält. Der Nadelschaft berührt die Einstichstelle nicht, wie aus dieser Darstellung ersichtlich ist, In dieser Lage wird durch das Vorwärtsschieben des Kolbens der Injektionsspritze 3 die Flüssigkeit mit gleichbleibendem Druck injiziert.

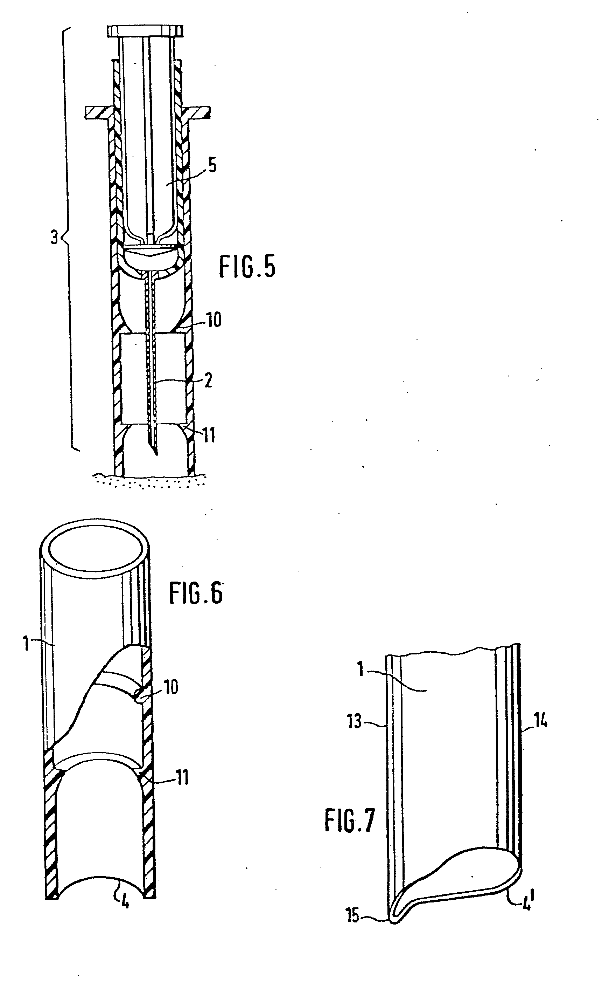

In Figur 5 ist eine Ausführungsform dargestellt, bei der die Injektionsnadel 2 und die Hülse integriert sind. Bei den neute üblichen Einwegspritzen besteht ohne wesentlicher Erhöhung der Herstellungskosten die Möglichkeit, die Hülse 1 an die Injektionsspritze 3 schon während der Fertigung anzuformen.

Figur 6 zeigt eine weitere Ausführungsform der Erfindung, bei der das Klemmelement 10 als entlang der Innenfläche der Hülse 1 umlaufender, deren Querschnitt verengender Wulst ausgebildet ist. Diese Ausführungsform ist besonders einfach herzustellen, da ein derartiger Wulst fertigungstechnisch sehr einfach an die Innenfläche der Hülse 1 angeformt werden kann. Die Erhabenheit des Wulstes kann 0,1 bis 0,5 mm betragen.

In Figur 7 ist eine weitere Ausführungsform der Hülse 1 perspektivisch dargestellt, wobei der obere Teil der Hülse 1 weggelassen wurde. Im Inneren ist diese Hülse genauso ausgebildet wie eine der in den Figuren 1 bis 3 bzw. in Figur 6 gezeigten Ausführungsbeispiele. Der Hülsenrand 4' verläuft gegenüber der Längsachse der Hülse 1 schräg und ist leicht gewellt ausgebildet, wobei der Hülsenrand 4' mit der einen Längsaußenkante 13 der Hülse 1 eine leicht nach unten gekrümmte Spitze 15 bildet und an der Stoßstelle mit der anderen Längsaußenkante 14 der Hülse 1 einen gleichmäßig abgerundeten Verlauf zeigt. Diese Ausführungsform ist insbesondere für eine Injizierung gedacht, bei der die Injektionsspritze 3 schräg angesetzt werden muß. Die nach unten gekrümmte Spitze 15 ermöglicht es, die Hülse 1 in ihrer Lage auf der Hautoberfläche genau zu fixieren, um den Einstich an der gewünschten Stelle vorzunehmen. Auch diese Ausführungsform kann mit einer Injektionsspritze zu einem einheitlichen Bauteil integriert werden.

**Patentansprüche**

1. Vorrichtung für Injektionsspritzen zur intra muskulären und subkutanen Injektion in der Human- und Veterinärmedizin, wobei unter Verwendung coaxial ineinander verschiebbarer Teile, einschließlich einem in Verlängerung und teilweiser Umfassung des Spritzenkörpers (5), unten zur Injektionsstelle hin offenen Applikator zum Ansetzen bzw. Applizieren an den zu behandelnden Körper (5), wobei durch Druck auf den Spritzenkörper dieser samt Nadel (2) unter Überwindung eines Klemmelementes` (10) zum raschen schmerzfreien Einstechen schlagartig nach vorne bewegbar ist bis zum Erreichen eines die Verschiebung aufhaltenden Anschlagelementes (11), dadurch gekennzeichnet, daß der mit einem Spritzenkörper (5) von gleichmäßigem Umfang zu kombinierende Applikator aus einer einteiligen im wesentlichen rohrförmigen Kunststoffhülse (1) besteht mit einem unteren für die Erzeugung eines Ablenkungsschmerzes geeigneten Rand (4) und einem diesem entgegengesetzt liegenden oberen Bereich, dessen Innendurchmesser etwas größer ist als der Außendurchmesser des von oben einzuführenden Spritzenkörpers (5), und daß die Hülse (1) weiterhin als Klemm

(10)—und Anschlagelement (11) an ihre Innenwand angeformte Erhebungen, insbesondere Wulste, als Querschnittsverengung aufweist in einem vorgegebenen axialen Abstand (d) voneinander, der der gewünschten Injektionstiefe zuzüglich einem Sicherheitsabstand zwischen Nadelspitze und unterem Hülsenrand (4) in Ausgangsstellung entspricht, wobei das Anschlagelement (11) zwischen Klemmelement (10) und unterem Hülsenrand (4) angeordnet ist.

2. Vorrichtung nach Anspruch 1, dadurch gekennzeichnet, daß das Klemmelement zumindest eine in Längsrichtung geschlitzte Zunge (6) mit einer nach innen weisenden Nase (7) besitzt.

3. Vorrichtung nach Anspruch 2, dadurch gekennzeichnet, daß die Hülse (1) zwei sich am Umfang der Hülse gegenüberliegende Zungen (6, 6') aufweist.

4. Vorrichtung nach einem der vorhergehenden Ansprüche, dadurch gekennzeichnet, daß das Anschlagelement (11) aus umlaufende Verengung im Inneren der Hülse ausgebildet ist.

5. Vorrichtung nach Anspruch 4, dadurch gekennzeichnet, daß die Hülse (1) aus einem nicht durchsichtigen Kunststoff besteht und daß das Anschlagelement (11) aus Wulst an die Innenfläche der Hülse angeformt ist.

6. Vorrichtung nach einem der Ansprüche 1 bis 5, dadurch gekennzeichnet, daß die Hülse (1) in Hähe des unteren Hülsenrandes (4) bzw im Bereich des Anschlagelementes (11) einen Zwischenboden (11a) aufweist, der eine zentrische Öffnung (9) zum Passieren der Injektionsnadel (2) besitzt.

7. Vorrichtung nach Anspruch 6, dadurch gekennzeichnet, daß der Zwischenboden (11a) mittig eine coaxiale Hülse (12) zur Führung und zum Schutz der Injektionsnadel (2) aufweist.

8. Vorrichtung nach einem der Ansprüche 1 bis 7, dadurch gekennzeichnet, daß das Klemmelement (10) als entlang der Hülse umlaufender, deren Querschnitt verengender Wulst ausgebildet ist.

9. Vorrichtung nach Anspruch 8, dadurch gekennzeichnet, daß der Wulst an die Innenwand der Hülse (1) angeformt ist und eine Erhabenheit von 0,1 bis 0,5 mm besitzt.

10. Vorrichtung nach einem der Ansprüche 1 bis 9, dadurch gekennzeichnet, daß der untere Hülsenrand (4') schräg zur Längsrichtung der Hülse (1) verläuft.

11. Vorrichtung nach Anspruch 10, dadurch gekennzeichnete, daß der Schräge Hülsenrand (4') einen leicht gewellten Verlauf aufweist, mit einer Längsaußenkante (13) der Hülse (1) eine leicht nach unten gekrümmte Spitze (15) bildet und an der Stoßstelle mit der anderen Längsaußenkante (14) der Hülse (1) gleichmäßig gekrümmt verläuft.

12. Vorrichtung nach einem der Ansprüche 1 bis 11, dadurch gekennzeichnet, daß die Hülse (1) mit der Injektionsspritze (3) integriert ist.

## Revendications

1. Dispositif pour seringues d'injection pour injection intramusculaire et sous-cutanée en médecine humaine et vétérinaire, comportant des parties pouvant coulisser coaxialement l'une dans l'autre, y compris un applicateur ouvert vers le bas en direction du point d'injection, dans le prolongement du corps de seringue (5) en entourant partiellement celui-ci, pour la pose ou l'application sur le corps à traiter (5), seringue dans laquelle, par pression sur le corps de seringue, celui-ci, et en même temps l'aiguille (2), peuvent se mouvoir brusquement vers l'avant en surmontant un élément de blocage (10), en vue d'une piqûre rapide et indolore, jusqu'à ce que soit atteint un élément de butée (11) arrêtant le coulissement, caractérisé par le fait que l'applicateur destiné à être combiné à un corps de seringue (5) de circonférence uniforme est formé d'une douille en matière synthétique en une seule partie (1), essentiellement tubulaire, munie d'un bord (4) convenant pour engendrer une douleur de distraction et d'une région supérieure située à l'opposé de celui-ci et dont le diamètre intérieur est un peu plus grand que le diamètre extérieur du corps de seringue (5) à introduire par le haut, et que la douille (1) présente en outre, comme rétrécissement de section, des saillies, en particulier des bourrelets, formés à sa paroi intérieure comme élément de blocage (10) et de butée (11), séparés par une distance axiale prescrite (d) qui correspond à la profondeur d'injection désirée plus un espacement de sécurité entre la pointe de l'aiguille et le bord inférieur (4) de la douille, en position initiale, l'élément de butée (11) étant disposé entre l'élément de blocage (10) et le bord inférieur (4) de la douille.

2. Dispositif selon la revendication 1, caractérisé par le fait que l'élément de blocage présente au moins unelanguette (6) fendue en direction longitudinale munie d'un bec (7) tourné vers l'intérieur.

3. Dispositif selon la revendication 2, caractérisé par le fait que la douille (1) présente deux languettes (6, 6') opposées entre elles à la circonférence de la douille.

4. Dispositif selon l'une des revendications précédentes, caractérisé par le fait que l'élément de butée (11) est conçu sous la forme d'un rétrécissement circonférentiel à l'intérieur de la douille.

5. Dispositif selon la revendication 4, caractérisé par le fait que la douille (1) est formée d'une matière synthétique non transparente et que l'élément de butée (11) est formé en tant que bourrelet à la surface intérieure de la douille.

6. Dispositif selon l'une des revendications 1 à 5, caractérisé par le fait que la douille (1) présente, au voisinage de son bord inférieur (4) ou dans la région de l'élément de butée (11), un fond intermédiaire (11a) qui présente une

ouverture centrée (9) pour le passage de l'aiguille d'injection (2).

7. Dispositif selon la revendication 6, caractérisé par le fait que le fond intermédiaire (11a) présente en son milieu une douille coaxiale (12) servant à guider et à protéger l'aiguille d'injection (2).

8. Dispositif selon l'une des revendications 1 à 7, caractérisé par le fait que l'élément de blocage (10) est conçu sous la forme d'un bourrelet faisant le tour de la douille et rétrécissant la section de celle-ci.

9. Dispositif selon la revendication 8, caractérisé par le fait que le bourrelet est formé sur la paroi intérieure de la douille (1) et présente un relief de 0,1 à 0,5 mm.

10. Dispositif selon l'une des revendications 1 à 9, caractérisé par le fait que le bord inférieur (4') de la douille est dirigé obliquement par rapport à la direction longitudinale de la douille (1).

11. Dispositif selon la revendication 10, caractérisé par le fait que le bord oblique (4') de la douille présente une allure légèrement ondulée, forme avec une arête extérieure longitudinale (13) de la douille (1) une pointe (15) légèrement courbée vers le bas et présente, au moint de raccordement avec l'autre arête longitudinale (14) de la douille (1), une courbure uniforme.

12. Dispositif selon l'une des revendications 1 à 11, caractérisé par le fait que la douille (1) est intégrée à la seringue d'injection (3).

**Claims**

1. A device for injection syringes for intramuscular and subcutaneous injection in human and veterinary medicine using parts shiftable coaxially within one another including an applicator which is open at the bottom and directed to the injection point and which in prolongation of the syringe body partly surrounds the body, for putting to or applying to the body to be treated, the syringes body (5) including the needle (2) for a quick and painless puncture being abruptly movable in a forward direction overcoming a clamping element (10) by applying pressure to the syringe body (5), until a stop element (11) is reached which limits the shifting movement, characterized in that the applicator to be combined with a syringe body (5) of a uniform circumference consist of a one-piece and substantially tubular sleeve (1) of synthetic material having a bottom edge (4) which is adapted for producing a diversionary pain and having an opposite top range the inside diameter of which being slightly larger than the outside diameter of the syringe body (5) to be inserted from the top, and that the sleeve (1) furthermore comprises as clamping (10) and stop (11) elements elevations, particularly beads formed on its inner wall and constricting its cross-section, the elements having a predetermined axial distance (d) from one another which distance corresponds to the desired injection depth plus a safety distance between the tip of the needle and the bottom edge (4) of the sleeve in the starting position, the stop element (11) being arranged between the clamping element (10) and the bottom edge (4) of the sleeve.

2. A device according to claim 1, characterized in that the clamping element has at least one tongue (6) which is slit in the longitudinal direction and has an inwardly extending nose (7).

3. A device according to claim 2, characterized in that the sleeve (1) has two tongues (6, 6') situated opposite one another on the sleeve periphery.

4. A device according to one of the preceding claims, characterized in that the stop element (11) is in the form of a continuous restriction in the interior of the sleeve.

5. A device according to claim 4, characterized in that the sleeve (1) consists on an opaque synthetic material and the stop element (11) is formed as a bead on the inner surface of the sleeve.

6. A device according to one of claims 1 to 5, characterized in that the sleeve (1) has an intermediate base (11a) near the bottom edge (4) of the sleeve or in the region of the stop element (11), said intermediate base having a central aperture (9) for the passage of the injection needle (2).

7. A device according to claim 6, characterized in that the intermediate base (11a) has a coaxial sleeve (12) centrally in order to guide and protect the injection needle (2).

8. A device according to one of claims 1 to 7, characterized in that the clamping element (10) is in the form of a bead extending round the sleeve and constricting its cross-section.

9. A device according to claim 8, characterized in that the bead is formed on the inner wall of the sleeve (1) and projects 0.1 to 0.5 mm.

10. A device according to one of claims 1 to 9, characterized in that the bottom edge (4') of the sleeve extends at an angle to the longitudinal direction of the sleeve (1).

11. A device according to claim 10, characterized in that the inclined edge (4') of the sleeve has a slightly corrugated configuration and cooperates with a longitudinal outer edge (13) of the sleeve (1) to form a slightly downwardly curved tip (15) while it extends with a uniform curvature at the junction point with the other longitudinal outer edge (14) of the sleeve (1).

12. A device according to one of claims 1 to 11, characterized in that the sleeve (1) is integral with the injection syringe (3).

FIG.1

FIG.2

FIG.3

FIG.4a

FIG.4b

FIG.4c

FIG.4d

FIG.4e

FIG.5

3

5

10

2

11

FIG.6

1

10

11

4

FIG.7

1

13

14

15

4'

3